Europäisches Patentamt

European Patent Office

Office européen des brevets

(19)

(11) Veröffentlichungsnummer: **0 120 406**

**A1**

# EUROPÄISCHE PATENTANMELDUNG

(12)

(21) Anmeldenummer: **84102754.3**

(22) Anmeldetag: **14.03.84**

(51) Int. Cl.³: **A 61 M 5/16**

(30) Priorität: **19.03.83 DE 8308079 U**

(43) Veröffentlichungstag der Anmeldung:
**03.10.84 Patentblatt 84/40**

(84) Benannte Vertragsstaaten:
**AT BE CH DE FR GB IT LI LU NL SE**

(71) Anmelder: **Büro für Infusionstechnik
Hauptstrasse 34
D-7550 Baden-Baden(DE)**

(72) Erfinder: **Strackharn, Klaus, Dr. med.
Forchheimerstrasse 30
D-8523 Baiersdorf(DE)**

(74) Vertreter: **Tergau, Enno et al,
Hefnersplatz 3 Postfach 11 93 47
D-8500 Nürnberg 11(DE)**

(54) Verfahren zur Überwachung einer Schwerkraft-Infusion sowie Vorrichtung zur Durchführung des Verfahrens.

(57) Bei einem Verfahren zur Überwachung einer Schwerkraftinfusion oder -Transfusion wird anfänglich durch ein elektrooptisches Zählgerät die Rate der einsetzenden Tropfenfolge in einem ersten Zeitintervall zahlenmäßig erfaßt, abgespeichert und angezeigt. Sodann wird der Zahlenwert der abgespeicherten Tropfrate mit dem Zahlenwert der Tropfrate in mindestens einem nachfolgenden Zeitintervall automatisch verglichen. Bei Übereinstimmung innerhalb vorgebbarer Toleranzen der Tropfraten mindestens zweier nacheinander abfolgender Zeitintervalle wird die als konstant ermittelte Tropfrate als Soll-Tropfrate elektronisch abgespeichert und/oder angezeigt.Bei Abweichung der fortlaufend automatisch durch das Zählgerät erfaßten Tropfraten während des gesamten Infusionsvorganges von der als konstant ermittelten und abgespeicherten Soll-Tropfrate wird durch das Zählgerät ein Warnsignal abgegeben.

FIG.1

EP 0 120 406 A1

0120406

3/23(84130)

**TERGAU & POHL**
PATENTANWÄLTE
HEFNERSPL. 3 · POSTF. 11 93 47
**8500 NÜRNBERG 11**

Büro für Infusionstechnik, Hauptstr. 34,

7550 Baden-Baden

<u>Verfahren zur Überwachung einer Schwerkraft-Infusion</u>

<u>sowie Vorrichtung zur Durchführung des Verfahrens</u>

Die Erfindung betrifft zunächst ein Verfahren zur Überwachung einer Schwerkraft-Infusion oder -Transfusion an einem Patienten.

Die Tropfrate einer Schwerkraftinfusion wird bei üblicher Vorgehensweise von Hand eingestellt, wobei die erforderliche Soll-Tropfenzahl/Zeiteinheit z.B. mittels einer Stoppuhr bestimmt und festgelegt wird. Der dafür erforderliche Zeitaufwand beträgt je nach Anforderung an die Genauigkeit der Einstellung mehrere Minuten. Eine weitere Überwachung des Infusionsverlaufes ist in der Regel nicht möglich, weil dies zusätzlichen Personalaufwand erfordern würde. Veränderungen der Tropfrate und damit der Dosierung bleiben in der Regel unbemerkt. Ebenso wird die Entleerung des Infusionsbehälters häufig nur zufällig entdeckt, wenn die Infusion längst durchgelaufen ist. In der Folge muß das Infusionsbesteck gewechselt, zumindest aber die darin eingeschleuste Luft entfernt werden. In nicht wenigen Fällen bereitet die Wiederaufnahme der Infusion erhebliche Schwierigkeiten, wenn nämlich der venöse Zugang am Patienten durch Blutgerinnsel verstopft ist. Dies ist um so häufiger zu beobachten, je länger der unbemerkte Flüssigkeitsstopp andauert.

Sämtliche Veränderungen der äußeren Bedingungen einer Infusion mit Ausnahme der definitiven Entleerung des Flüssigkeitsbehälters sowie der Diskonnexion des Infusionsschlauches führen innerhalb nicht vorhersehbarer Zeiträume zu einem Infusionsstopp. Dabei kommt es zunächst zu einer allmählichen Reduzierung der Tropfrate, wodurch eventuelle Gerinnungsprozesse in der Patientenader gefördert werden, die dann ihrerseits den endgültigen Infusionsstopp auslösen.

Von diesen Vorgängen abgesehen hat jede größere und länger andauernde Abweichung der voreingestellten Tropfrate nachteilige Folgen für den Patienten. Sei es, daß z.B. die Destabilisierung wichtiger Kreislauffunktionen eintritt oder daß das Auftreten von Verwertungsstörungen begleitet von Übelkeit, Erbrechen und Durchfall erfolgt oder durch die Umverteilung lebenswichtiger Mineralsalze schwere Regulationsstörungen ausgelöst werden. Als Folge dieser unerwünschten Nebenwirkungen werden zusätzliche intensivtherapeutische Maßnahmen erforderlich. Daher wird allgemein die Überwachung der eingestellten Infusionstropfrate möglichst innerhalb vorwählbarer Grenzen gefordert.

Der Erfindung liegt die Aufgabe zugrunde, zunächst ein Verfahren anzugeben, das eine vollständige Überwachung des gesamten Infusionsablaufes gewährleistet, wobei bereits unmittelbar nach Infusionsbeginn eine rasche Orientierung über die eingestellte Tropfrate, sodann eine Überwachung auf Infusionsstopp einschließlich der Entleerung des Infusionsbehälters sowohl auf unzulässig hohe Förderrate und zusätzlich auf verschiedene vorwählbare Toleranzen möglich sein soll. Diese Aufgabe wird durch die Kombination der kennzeichnenden Merkmale des Anspruches 1 gelöst.

Durch die wohlgeordnete Abfolge der Merkmale des Verfahrensanspruches 1 hat die Bedienungsperson die Möglichkeit, im Zusammenhang mit dem das Verfahren durchführenden Gerät sich zunächst sehr schnell ohne Zuhilfenahme einer gesondert abzulesenden Stoppuhr über die aktuelle Tropfrate zu informieren. Sodann wird als nächster Verfahrensschritt festgestellt, ob die (eingestellte und angezeigte) Tropfrate in gewissen Toleranzen konstant ist. Falls dies verfahrensmäßig erkannt wird, wird die "konstante" Tropfrate erneut abgespeichert und deren Einhaltung während des gesamten Infusionsverlaufes innerhalb vorgebbarer Toleranzgrenzen überprüft. Durch das Verfahren können auf einfache Weise somit folgende Situationen erfaßt werden:

- Entleerung des Infusionsbehälters,
- abgeknickte Infusionsleitung,
- Verlegung des Zuflusses durch Gerinnselbildung in der Patienten-Vene,
- Verlegung des Zuflusses durch Irritation der Punktionskanüle bzw. des Katheters,
- Endstadium einer "Vena-Para-Infusion", d.h. einer Flüssigkeitszufuhr in die die Patientenader umgebenden Gewebe, nachdem die Punktionskanüle die Venenwand durchstochen hat,
- erhebliche Veränderungen des hydrostatischen Potentials zwischen Flüssigkeitsspiegel zum Infusionsbehälter und Punktionsort bzw. Katheteröffnung,
- unbefugte Manipulation an der Dosierklemme des Infusionsschlauches,
- Diskonektion des Infusionsschlauches oder einer Steckverbindung,
- herausgerissene Punktionskanüle, herausgerissener Katheter.

Mit dem Verfahren ist es somit möglich, die häufigsten Komplikationen während der Durchführung einer Infusion in Griff zu bekommen, ohne daß dazu ein übermäßiger Personalaufwand notwendig ist.

Jenachdem, welcher Art die dem Patienten durch Infusion applizierte Substanz ist, können für die Überwachung unterschiedliche Toleranzbereiche vorgegeben werden, bei deren Überschreiten verfahrensgemäß Alarm ausgelöst wird.

Ansprüche 3 ff. betreffen eine Vorrichtung zur Durchführung des Verfahrens nach Anspruch 1 oder Anspruch 2, mit welcher das Verfahren besonders vorteilhaft und sicher durchgeführt werden kann. Im einzelnen besteht die Vorrichtung aus einer mit einer optischen Strahlsensoreinheit zusammenwirkenden Auswerteelektronik, der eine Anzeigevorrichtung sowie ein Alarmgeber nachgeschaltet sind. Sämtliche Komponenten sind in einem speziell ausgebildeten Gehäuse untergebracht.

Durch die Merkmale des Anspruches 3 wird zunächst bei Infusionsbeginn die aktuelle Tropfrate, d.h. die Anzahl der Tropfen pro Zeiteinheit, die durch einen Zeitgeber vorgewählt werden kann, in einen ersten Zähler eingegeben, dessen Eingang der Sensoreinheit der optischen Strahlsendeeinheit nachgeschaltet ist. Der aktuelle Stand dieses Zählers wird nach Beendigung des Zeitintervalls, d.h. beispielsweise nach 8 Sekunden, immer wieder erneut angezeigt und gibt somit der Bedienungsperson einen Überblick über die aktuelle Tropfrate.

Sodann wird der Zählerstand mit dem Inhalt eines ersten Speichers verglichen, der die Tropfraten in bereits abgelaufenen Zeitintervallen wiederspiegelt. Wird durch einen dem Speicher und Zähler nachgeschalteten Komparator erkannt, daß sich eine Tropfrate mit gewisser Konstanz eingestellt hat, so wird diese als Sollwert in einem weiteren Speicher abgelegt und während nachfolgender Zeitabschnitte (diese werden auch durch den Zeitgeber vorgegeben) auf Abweichung nach oben oder unten innerhalb gewisser Toleranzgrenzen überwacht. Die Toleranzgrenzen sind über einen an dem Gehäuse der Vorrichtung angeordneten Wählschalter einstellbar. Erkennt der Komparator, daß die Abweichung vom Sollwert zu groß wird, so wird eine Rufvorrichtung in Form eines Summers, Lichtsignals o.dgl. aktiviert. Auch ist es möglich, beispielsweise eine Schwesternrufanlage einzuschalten, die über eine elektrische Steckverbindung an das Gerät angeschlossen werden kann.

Gemäß Anspruch 4 weist die Setzvorrichtung eine optische Anzeigevorrichtung (in Form eines Lämpchens o.dgl.) auf, das so lange leuchtet, bis die Vorrichtung eine Tropfenrate mit einer gewissen Mindestkonstanz detektiert hat. Zudem ist eine Setztaste vorgesehen, die elektronisch erst dann freigegeben wird, wenn die optische Anzeigevorrichtung erlischt und damit die Bedienungsperson weiß, daß sich die Tropfrate stabilisiert hat.

Vorteilhafterweise sind alle Komponenten der Vorrichtung in einem gemeinsamen Gehäuse untergebracht (abgesehen von einem Netzteil). Das Gehäuse ist balkenförmig aufgebaut und beinhaltet auf der einen Seite die optische Strahlsendeeinheit und auf der anderen Seite die Sensoreinheit mit der nachfolgenden Auswerteelektronik und Anzeigeeinheit. Mittig umfaßt das Gehäuse z.B. die Tropfkammer eines Infusionsbesteckes derart, daß der Strahl der optischen Strahlsendeeinheit durch die Tropfenbahn hindurchsteht. Ein derartiges Kompaktgerät ist ohne großen Aufwand zum Patienten transportierbar und dort auf einfache Weise einsetzbar.

Eine herausragende Bedeutung hat der Einsatz des Gerätes für alle Schwerkraft-Infusionen bzw. -Transfusionen, vor allem aber dort, wo Personal eingespart werden muß oder nicht mehr vorhanden ist, wie während der Nacht.

Als Sensoreinheit innerhalb des Gehäuses kommen eine Fotozelle, eine Fotodiode oder ein Fototransistor in Betracht. Die Sensoreinheit kann auf kürzestem Wege mit der Auswerteelektronik gekoppelt werden, die als Mikroprozessor ausgebildet sein kann. Die Funktionen können darin bestehen, die durchschnittliche Tropfenfolge anzugeben, die sich über die Zeit ändern kann. Die Funktion kann darüber hinaus darin bestehen, die Abweichung der Tropfenfolgen in Prozenten von einer Solltropfenfolge anzugeben, wobei verschiedene Toleranzbereiche einstellbar sind. Beim Einsatz eines Mikrocomputers ist es desgleichen möglich, bereits am Anfang der Messung durch eine Hochrechnung die sich letztendlich einstellende Tropfenfolge je Zeiteinheit anzugeben. Es hat dies den erheblichen Vorteil, daß die am Tropfgerät zu beobachtende Tropfenfolge bereits nach wenigen Sekunden angezeigt wird, so daß Nachregulierungen entsprechend schnell vorgenommen werden können.

Eine unzulässige Störung der Tropfenfolge liegt auch dann vor, wenn es wegen einer Knickbildung im Schlauch oder einer Gerinnselbildung in der Blutader des Patienten oder durch Entleerung des Infusionsbehälters zum Stillstand des Flüssigkeitstransportes gekommen ist oder wenn die tropfende Infusionsflüssigkeit in einen ununterbrochenen Strahl übergegangen ist, z.B. infolge einer Loslösung des Infusionsschlauches von der in der Patientenader liegenden Kanüle. Die erwähnte akustische und/oder optische Warneinrichtung kann dabei entweder im erwähnten Kompaktgehäuse oder auch in einem mit ihm verbundenen Netzanschlußteil eingebaut sein. Schließlich ist es auch möglich, einen Steckeranschluß vorzusehen, über den ein weiterer Signalgeber, z.B. eine Schwesternrufanlage, an das Gerät angeschlossen werden kann.

Die Erfindung ist anhand eines Ausführungsbeispieles näher erläutert. Es zeigen:

Fig. 1 ein vereinfachtes Blockschaltbild der Auswerte elektronik,

Fig. 2 eine perspektivische Darstellung des Gehäuses der Vorrichtung.

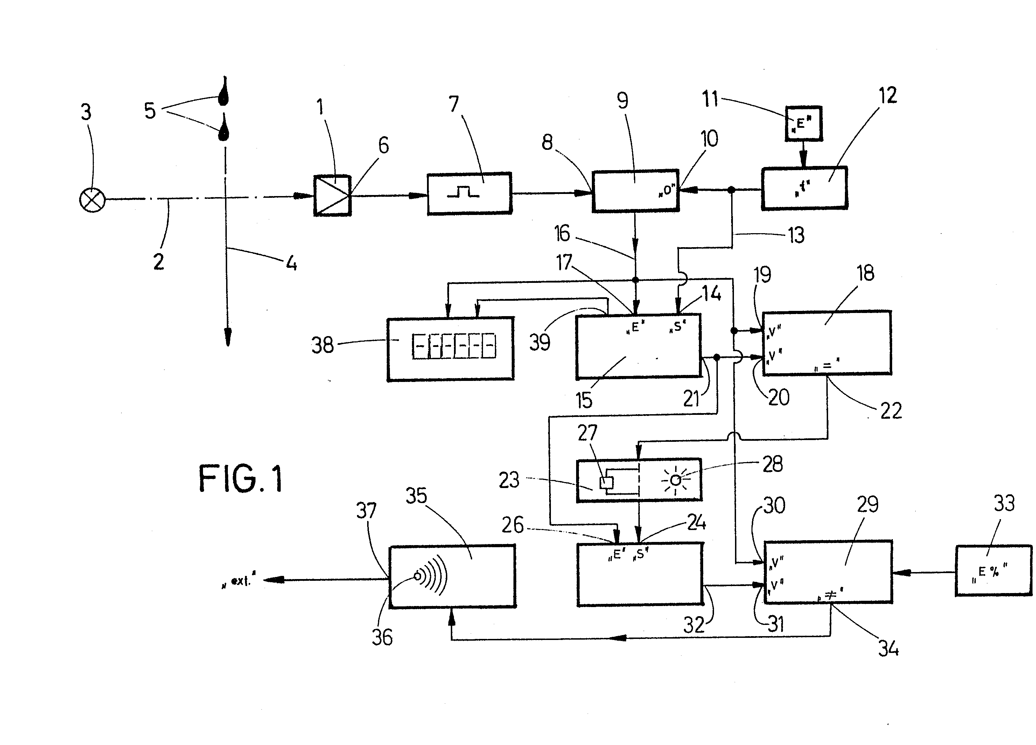

Die in Fig. 1 als vereinfachtes Blockschaltbild dargestellte Auswerteelektronik besteht zunächst aus einer Sensoreinheit 1, die den Lichtstrahl 2 einer optisches Strahlsendeeinheit (Lampe 3) detektiert, der den Tropfenweg 4 kreuzt und somit durch die hindurchgehenden Tropfen 5 unterbrochen oder zumindest moduliert wird, daß ein Spannungssignal am Ausgang 6 der Sensoreinheit 1 vorliegt.

In einer dem Ausgang 6 der Sensoreinheit 1 nachgeschalteten Impulsformerstufe 7 werden die Spannungssignale derart aufbereitet, daß sie auf den Eingang 8 eines Zählers 9 gegeben werden können. Der Nullungseingang 10 des Zählers 9 ist mit einem über eine Eingabevorrichtung 11 setzbaren Zeitglied 12 verbunden, so daß nach Ablauf der voreingestellten Zeit der Zähler 9 auf Null gesetzt wird und erneut zu zählen beginnt. Vor dem Nullsetzen wird über eine Leitung 13 ein Übernahmebefehl auf den Setzeingang 14 eines dem Zähler 9 nachgeschalteten ersten Speichers 15 abgegeben, so daß der während des voreingestellten Zeitintervalles im Speicher 9 aufgelaufene Zählwert über die Leitung 16 an den Zähleingang 17 des ersten Speichers 15 abgegeben und dort abgespeichert wird.

Die Vergleichseingänge 19,20 eines ersten Komparators 18 sind mit dem Ausgang des Zählers 9 bzw. mit dem Ausgang 21 des ersten Speichers 15 verbunden. Der erste Komparator vergleicht somit laufend, ob der Zählerstand pro voreingestellter Zeiteinheit (Tropfrate) in einem zweiten Zeitintervall mit der im Speicher 15 abgelegten Tropfrate eines vorhergehenden ersten Zeitintervalles übereinstimmt. Falls der erste Komparator 18 die Übereinstimmung feststellt, wird über den Komparatorausgang 22 eine Freigabevorrichtung 23 angesteuert, die einen Setzbefehl an den Setzeingang 24 eines zweiten Speichers 25 abgibt, so daß der im Speicher 15 als konstante Tropfrate abgelegte Speicherwert über den Eingang 26 im zweiten Speicher 25 abgelegt wird. Der Setzbefehl kann zum Setzeingang 24 des zweiten Speichers entweder unmittelbar durch die Freigabevorrichtung erfolgen, es ist jedoch auch möglich, eine gesonderte Setztaste 27 vorzusehen, die wirkungsmäßig erst dann freigegeben wird, wenn der erste Komparator 18 das Vorliegen gleicher Werte an den Vergleichseingängen 19,20 erkennt. Über die Setztaste 27 hat die Bedienungsperson die Möglichkeit, den Setzbefehl manuell auszulösen. Eine Leuchtanzeige 28 wirkt mit der Freigabevorrichtung 23 zusammen und leuchtet, solange die Freigabe der Setztaste 27 noch nicht erfolgt ist.

In einem zweiten Komparator, dessen Vergleichseingänge 30,31 mit dem Ausgang des Zählers 9 bzw. dem Ausgang 32 des zweiten Speichers 25 verbunden sind, wird nun überwacht, ob die vom Zähler 9 laufend während des Infusionsvorganges ermittelte Zählrate mit der im zweiten Speicher 25 abgelegten Sollzählrate übereinstimmt. Falls der zweite Komperator 29 eine Abweichung erkennt, die über eine durch eine Eingabevorrichtung 33 voreinstellbare Toleranzgrenze (z.B. 50%, 20%, 10% tolerierbare Abweichung) hinausgeht, wird von dem Ausgang 34 ein Signal an eine Rufvorrichtung 35 abgegeben, die mit einer Signalquelle 36 optisch oder akustisch anzeigt, daß der Infusions- oder Transfusionsvorgang nicht ordnungsgemäß abläuft. Die Rufvorrichtung 35 weist zudem einen Steckeranschluß 37 auf,
der den Anschluß beispielsweise einer krankenhausinternen Schwesternrufanlage ermöglicht.

Eine Anzeigeeinheit 38 ist mit dem Ausgang des Zählers
9, mit einem weiteren Ausgang 39 des ersten Speichers
15 sowie ggf. mit weiteren Elementen, beispielsweise
dem Zeitglied 12, verbunden, um die Zähler- oder
Speicherwerte anzuzeigen.

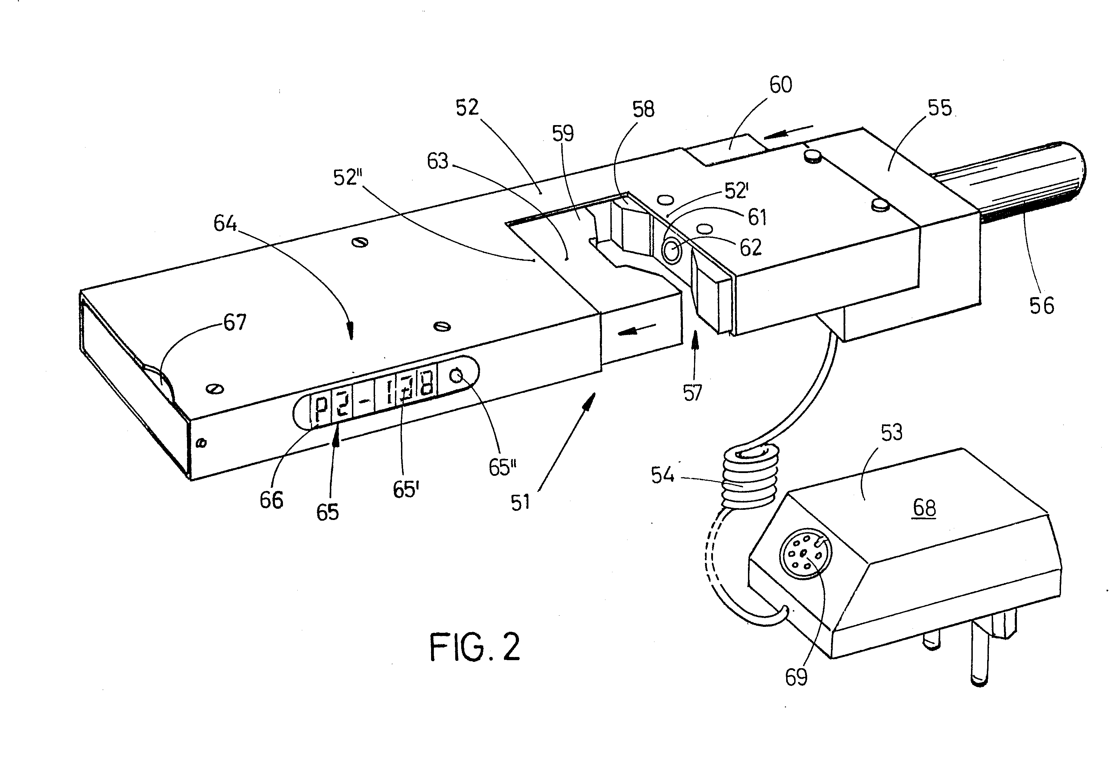

Das in Fig. 2 dargestellte Überwachungs- und Anzeigegerät ist als Ganzes mit 51 bezeichnet. Es weist ein
balkenförmiges Gehäuse 52 sowie - im dargestellten
Ausführungsbeispiel - ein Netzanschlußteil 53 auf, die
mittels einer Verbindungsschnur 54 elektrisch miteinander verbunden sind.

Das Gehäuse 52 ist des weiteren noch mit einem
Montageblick 55 verbunden, der zum Befestigen des
Gehäuses, z.B. über einen Haltearm 56, an einem in der
Zeichnung nicht dargestellten Stativ dient. Der Montageblock 55 kann auch in anderer Weise konstruktiv mit
dem Gehäuse verbunden oder auch in dieses eingebaut
sein.

Das Gehäuse 52 ist im dargestellten Ausführungsbeispiel
U-förmig gestaltet und umschließt die Klemmvorrichtung
57 mit seinen Schenkeln 52' und 52''. Mit der
Klemmvorrichtung 57 wird beispielsweise die Tropfkammer
eines Infusionsbesteckes umklammert und so gegenüber
den Sende- und Empfangsteilen des Gerätes festgelegt.
Die Klemmvorrichtung 57 besteht aus einer festen
Klemmbacke 58 sowie einer gegen Federkraft zurückschiebbaren beweglichen Klemmbacke 59. Die bewegliche Klemmbacke kann am Griffteil 60 zurückgeschoben werden. Im Innern beider Klemmbacken befinden sich elektronische Bauelemente. So ist in der beweglichen Klemmbacke 59 eine optische Strahlsendeeinheit 61 z.B. ein Infrarotsender mit den dazu möglicherweise erforderlichen Hilfselementen eingebaut, in der gegenüberliegenden Klemmbacke 58 befindet sich die Sensoreinheit 63 mit nachfolgendem Mikrocomputer 64, Anzeigenblock 65 sowie Programmschalter 66.

Die optische Strahlsendeeinheit 61 sendet bei Betrieb einen Meßstrahl aus der in der Zeichnung erkennbaren Strahlenöffnung 62. Der Meßstrahl passiert im Betriebsfalle die schon erwähnte Tropfkammer und fällt alsdann auf die in der Zeichnung nur der Lage nach angedeutete Sensoreinheit 63. Hier erzeugt er entweder ein Dauersignal oder, wenn er durch fallende Tropfen rhythmisch unterbrochen wird, eine entsprechende Impulsfolge. Die Sensoreinheit 63 ist mit dem Mikrocomputer 64 und dieser mit dem Anzeigeblock 65 verbunden, letzter besteht aus mehreren alpha-numerischen Anzeigen sowie einer Anzeigelampe und ist hinter einem Sichtfenster 66 angeordnet. Im Gehäuse 52 befindet sich des weiteren noch der Programmschalter 67, mit welchem alle Überwachungsfunktionen und Betriebszustände eingestellt werden können.

Des weiteren kann sich im Gehäuse 52 oder aber auch im Netzanschlußteil 53 eine Rufvorrichtung 68 in Form eines Summers, eines piezoelektrischen Signalgebers o.dgl. befinden. Parallel dazu kann im Gehäuse 52 oder im Netzanschlußteil 53 ein Steckanschluß 69 angeordnet sein, über den das Gerät an einen weiteren Signalgeber, beispielsweise eine Schwesternrufanlage, angeschlossen werden kann.

Das vorgeschlagene Überwachungs- und Anzeigegerät kennzeichnet sich durch geringe Abmessungen, geringes Gewicht sowie Preiswürdigkeit aus. Es kann bequem in allen in Betracht kommenden Räumen einschließlich Hilfs- und Rettungsfahrzeugen mitgeführt und montiert werden. Das Gerät eignet sich vor allem zur Benutzung am Krankenbett in all den Fällen, in denen eine Infusionsüberwachung notwendig ist, eine hochgenaue Medikamenten- oder Flüssigkeitsdosierung mit Hilfe größerer Überwachungsapparate mit energetischem Antrieb jedoch nicht erforderlich ist oder aber derartige Geräte nicht zur Verfügung stehen.

0120406

3/23(84130)

**TERGAU & POHL**
PATENTANWÄLTE
HEFNERSPL 3  POSTF. 11 93 47
8500 NÜRNBERG 11

Büro für Infusionstechnik, Hauptstr. 34,

7550 Baden-Baden

Patentansprüche

1. Verfahren zur Überwachung einer Schwerkraftinfusion oder -transfusion an einem Patienten, gekennzeichnet durch die Kombination folgender Merkmale:

- Bei Beginn der Infusion (Transfusion) wird durch ein elektrooptisches Zählgerät die Rate der einsetzenden Tropfenfolge in einem ersten Zeitintervall zahlenmäßig erfaßt, abgespeichert und angezeigt,

- der Zahlenwert der abgespeicherten Tropfrate wird mit dem Zahlenwert der Tropfrate in einem oder mehreren nachfolgenden Zeitintervallen automatisch verglichen,

- bei Übereinstimmung innerhalb vorgebbarer Toleranzen der Tropfraten zweier oder mehrerer nacheinander abfolgender Zeitintervalle wird die als konstant ermittelte Tropfrate als Soll-Tropfrate elektronisch abgespeichert und/oder angezeigt,

- bei Abweichung der fortlaufend automatisch durch das Zählgerät erfaßten Tropfraten während des gesamten Infusionsvorganges von der als konstant ermittelten und abgespeicherten Soll-Tropfrate wird durch das Zählgerät ein Warnsignal abgegeben.

2. Verfahren nach Anspruch 1,

dadurch gekennzeichnet,

daß zur Auslösung des Warnsignales bei Abweichung der fortlaufend gezählten Tropfraten ein oder mehrere Toleranzbereiche vorwählbar sind.


3. Vorrichtung zur Durchführung des Verfahrens nach Anspruch 1 oder 2,

gekennzeichnet durch folgende Merkmale:

- Mit einer Sensoreinheit (1), die mit dem Lichtstrahl (2) einer die Tropfenbahn durchsetzenden optischen Strahlsendeeinheit (Lampe) zusammenwirkt, ist ein mit einem Zeitglied (12) zusammenwirkender Zähler verbunden, der die aktuelle Tropfrate als Tropfen/Zeiteinheit zahlenmäßig erfaßt,

- mit einem Zählerausgang ist eine Anzeigeeinheit (38) sowie der Eingang (17) eines ersten Speichers (15) verbunden, der den in einem ersten durch den Zeitgeber (12) bestimmten vorhergehenden Zeitintervall ermittelten Zählerstand erfaßt,

- die Ausgänge des Zählers (9) und des ersten Speichers (15) sind mit den Vergleichseingängen (19,20) eines ersten Komparators (18) zur Feststellung der Übereinstimmung der Inhalte des Zählers (9) und des ersten Speichers (15) innerhalb vorwählbarer Toleranzgrenzen verbunden,

- dem Zähler (9) oder dem ersten Speicher (15) ist über eine Setzvorrichtung (Setztaste 27) der Eingang (26) eines weiteren Speichers (25) zur Erfassung des Sollwertes nachgeschaltet, wobei die Setzvorrichtung (Setztaste 27) durch den ersten Komparator (18) bei Übereinstimmung der Inhalte des Zählers (9) und des ersten Speichers (15) aktivierbar ist,

- mit dem Ausgang (32) des zweiten Speichers (25) ist ein erster Vergleichseingang (31) eines zweiten Komparators (29) verbunden, dessen zweiter Vergleichseingang (30) dem Ausgang des Zählers (9) nachgeschaltet ist und der bei Überschreitung vorgebbarer Toleranzen zwischen dem Inhalt des zweiten Speichers (25) und dem Stand des Zählers (9) eine nachgeschaltete Rufvorrichtung (35) auslöst.

4. Vorrichtung nach Anspruch 3,

        dadurch gekennzeichnet,

daß die Setzvorrichtung mit einer manuell betätigbaren Setztaste (27) sowie einer optischen Anzeigevorrichtung (Leuchtanzeige 28) versehen ist, welch letzte die Sperrung der Setztaste (27) bei Abweichung des Inhaltes des ersten Speichers (15) vom Stand des Zählers (9) anzeigt.

5. Vorrichtung nach Anspruch 3 oder 4,

        dadurch gekennzeichnet,

daß die Rufvorrichtung (35) einen Steckanschluß (37) für einen weiteren Signalgeber (Schwesternrufanlage) aufweist.

6. Vorrichtung nach einem der vorhergehenden Ansprüche, dadurch gekennzeichnet,

daß die Speicher (15,25), der Zähler (9), der Zeitgeber (17), die Komparatoren (18,29) und die Setzvorrichtung Bestandteile eines mit der Lichtschranke (Sensoreinheit 1, Lampe 3) und der Anzeigeeinheit (38) in einem gemeinsamen Gehäuse (52) untergebrachten Mikrocomputers (64) sind.

7. Vorrichtung nach einem der vorhergehenden Ansprüche,

        dadurch gekennzeichnet,

daß an dem Gehäuse (52) ein Programmschalter (67) zur Eingabe der erlaubten Toleranzgrenzen angeordnet ist.

8. Vorrichtung nach einem der vorhergehenden Ansprüche, dadurch gekennzeichnet,

daß das Gehäuse (52) ein balkenförmiges Klemmgehäuse mit integrierter Klemmvorrichtung (57) ist, auf deren einer Seite sich die optische Strahlsendeeinheit (61) und auf deren anderer Seite sich die Sensoreinheit (51) mit nachfolgendem Zähler (9), Speichern (15,25), Komparatoren (18,29) (Auswerteelektronik) und Anzeigeeinheit (38) befindet.

9. Vorrichtung nach einem der vorhergehenden Ansprüche, dadurch gekennzeichnet,

daß das Gehäuse (52) U-förmig um die Klemmvorrichtung (57) gebaut ist, wobei sich in dem einen Schenkel die optische Strahlsendeeinheit (61) mit Hilfsgeräten und im anderen Schenkel die Sensoreinheit (63) mit Auswerteelektronik, Anzeigeeinheit (38) sowie Programmschalter (67) befinden.

10. Vorrichtung nach einem der vorhergehenden Ansprüche,

dadurch gekennzeichnet,

daß in einem Sichtfenster ein Anzeigenblock (65) der von der Auswerteelektronik ausgegebenen Signale für die errechnete Tropfrate, die angewählte Programmstufe sowie verschiedene Alarme vorhanden ist.

11. Vorrichtung nach einem der vorhergehenden Ansprüche,

dadurch gekennzeichnet,

daß in das Gehäuse (52) oder in einem mit diesem verbundenen Netzanschlußteil (53) die Rufvorrichtung (68) in Form eines Summers o.dgl. eingebaut ist.

12. Vorrichtung nach einem der vorhergehenden Ansprüche,

dadurch gekennzeichnet,

daß sich in einem Montageblock (55) eine Vorrichtung zur Aufnahme eines Haltearms (56) zur im wesentlichen horizontalen Befestigung der Strahlsendeeinheit (61) an einem Stativ befindet.

FIG.1

FIG. 2

## EINSCHLÄGIGE DOKUMENTE

| Kategorie | Kennzeichnung des Dokuments mit Angabe. soweit erforderlich, der maßgeblichen Teile | Betrifft Anspruch | KLASSIFIKATION DER ANMELDUNG (Int. Cl. 3) |
|---|---|---|---|
| A | GB-A-2 033 618 (NOUSIN HO) <br> * Seite 1, Zeilen 73-84; Seite 1, Zeile 90 - Seite 2, Zeile 104; Zeichnung * <br><br> --- | 1,3 | A 61 M 5/16 |
| A | FR-A-2 281 601 (UNION CHIMIQUE CONTINENTALE) <br> * Seite 5, Zeilen 6-35; Zeichnung 1 * <br><br> ----- | 1,3 | |

RECHERCHIERTE SACHGEBIETE (Int. Cl. 3)

A 61 M
G 01 F
G 01 P

Der vorliegende Recherchenbericht wurde für alle Patentansprüche erstellt.

| Recherchenort | Abschlußdatum der Recherche | Prüfer |
|---|---|---|
| DEN HAAG | 22-05-1984 | KNAUER F.E. |